# EUROPÄISCHE PATENTSCHRIFT

(11) **EP 3 297 982 B1**
(45) Veröffentlichungstag und Bekanntmachung des Hinweises auf die Patenterteilung: **03.07.2019**
(21) Anmeldenummer: 16727324.2
(22) Anmeldetag: 19.05.2016
(51) Int. Cl.: C07C 29/132, C07C 35/17, C07C 35/18, C07C 407/00, C07C 409/14

(54) **OXIDATION VON LIMONEN**
OXIDATION OF LIMONENE
OXIDATION DE LIMONÈNE

(30) Priorität: 21.05.2015 EP 15168597
(43) Veröffentlichungstag der Anmeldung: 28.03.2018
(73) Patentinhaber: Symrise AG, 37603 Holzminden (DE)
(72) Erfinder: DILK, Erich, 37603 Holzminden (DE); GEISEL, Detlef, 37603 Holzminden (DE); LAMBRECHT, Stefan, 37619 Hehlen (DE)
(74) Vertreter: Fabry, Bernd
(86) Internationale Anmeldenummer: PCT/EP2016/061232
(87) Internationale Veröffentlichungsnummer: WO 2016/184953

(56) Entgegenhaltungen:
- WO-A2-2009/033247
- US-A- 3 014 047
- GÜNTHER O. SCHENCK ET AL: "Zur chemischen und sterischen Selektivität der photosensibilisierten O2-Übertragung auf (+)-Limonen und (+)-Carvomenthen", JUSTUS LIEBIGS ANNALEN DER CHEMIE, Bd. 674, Nr. 1, 9. Juli 1964 (1964-07-09), Seiten 93-117, XP055224411, WEINHEIM; DE ISSN: 0075-4617, DOI: 10.1002/jlac.19646740111 in der Anmeldung erwähnt

## Beschreibung

### GEBIET DER ERFINDUNG

Die Erfindung betrifft ein Verfahren zur Herstellung von oxidierten Derivaten von Limonen, insbesondere von Epoxy- und Peroxid-Derivaten, und ganz besonders bevorzugt Peroxid-Derivaten, sowie die Verwendung solcher Verbindungen zur Herstellung von Duft-, Aroma- oder Geschmacksstoffen bzw. die Verwendung solcher Verbindungen als Zwischenprodukten zur Herstellung von Duft-, Aroma- oder Geschmacksstoffen.

### STAND DER TECHNIK

Der Wert von ätherischen Ölen als Duft-, Aroma- oder Geschmacksstoffe ist längst bekannt. Die aromatischen Hauptkomponenten von ätherischen Ölen aus Zitrusfrüchten sind Monoterpene, Sesquiterpene und deren sauerstoffhaltige Derivaten. Die sensorischen Eigenschaften von Zitrusaromen hängt hauptsächlich von dem Gehalt an sauerstoffhaltiger Terpene-Derivaten, Alkohole, Aldehyde, Ester und Ketonen ab.

Es ist auch bekannt, dass Monoterpene, insbesondere Limonen, wegen Ihrer hohen Konzentration die sensorischen Eigenschaften von Zitrusaromen negativ beeinflussen. Demzufolge sind verschiedenen Verfahren zur Abtrennung von Limonen entwickelt worden.

Limonen ist eine chemische Verbindung, die in der Natur vorkommt und der Gruppe der Terpene zugeordnet wird.

Beispielweise offenbart Fan et al in J. Agric. Food Che., 2004, 52(16), 5162-5167 die Abtrennung von Monoterpenen, unter anderem Limonen, durch die Kombination von konventioneller Vakuumdestillation mit superkritischen Kohlenstoffdioxid Extraktion.

Eine andere Möglichkeit ist Limonen in wertvolle Verbindungen zu verwandeln, die selbst wertvolle Duft-, Aroma- oder Geschmacksstoffe sind, bzw. in Zwischenverbindungen, die in verschiedensten Duft-, Aroma- oder Geschmacksstoffen weiter verarbeitet werden können. So ist es allgemein bekannt, dass Limonen als Edukt für die Herstellung von bestimmten Duft-, Aroma- oder Geschmacksstoffen oder auch deren Zwischenverbindungen dient.

Im Rahmen unserer Suche nach wertvollen Duft-, Aroma- oder Geschmacksstoffen haben sich oxidierte Derivaten von Limonen, insbesondere Epoxy- und Peroxid-Derivaten, und ganz besonders bevorzugt Peroxid-Derivaten, als besonders interessante Verbindungen gezeigt. Beispielweise seien die Verbindungen 1-6 genannt:

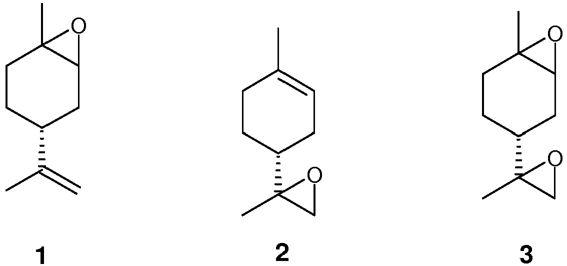

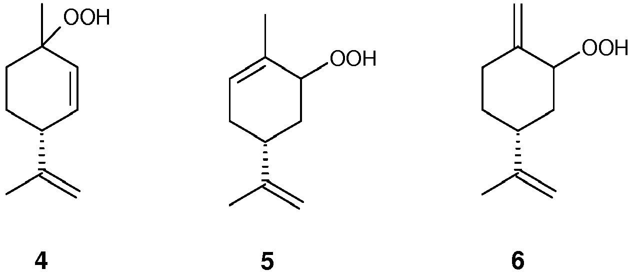

Ein bekanntes Verfahren zur Herstellung von Peroxid-Derivaten des Types der Verbindungen 4-6 umfasst die photochemische Oxidation von Limonen in Gegenwart von Wasserstoffperoxid (H₂O₂). Das dabei intermediär gebildete Hydroperoxid-Gemisch wird durch Behandlung mit Natriumsulfit-Lösung in das entsprechende Alkoholgemisch überführt (z.B. G .O. Schenk et al, Liebigs Ann. Chemie, 674 (1964), 93-117**).**

Der Nachteil photochemischer Herstellverfahren besteht darin, dass diese in speziellen Photoreaktoren durchgeführt werden. Diese Spezialausrüstungen erfordern im Vergleich zu üblichen chemischen Reaktoren höhere Investitionen und sind daher auch weniger verbreitet. Der Betrieb von Photoreaktoren ist zudem aufwändig.

In J. Am. Chem. Soc., 1968,90,975 wird eine Singlet Sauerstoff Oxidation (¹O₂-Ox) beschrieben, bei welcher ¹O₂ nicht photochemisch sondern chemisch generiert wird. Dabei werden hydrophobe Substrate mittels eines Hypochlorit/H₂0₂-Systems in einem Lösungsmittelgemisch aus Wasser und organischem Lösungsmittel oxidiert. Dieses Verfahren hat jedoch lediglich einige synthetische Anwendungen gefunden, da viele Substrate in dem benötigten Medium nur schwer löslich sind. Die Einsatzmöglichkeit ist außerdem aufgrund von Nebenreaktionen zwischen Hypochlorit und Substrat oder Lösungsmittel ziemlich eingeschränkt. Außerdem wird in der Gasphase ein großer Teil des ¹O₂ deaktiviert. Weiteres ist dieses Verfahren nicht für den industriellen Maßstab geeignet, da es im organischen Medium zur Anlagerung des Hypochlorits an H₂0₂ kommt und ein großer Überschuss an H₂0₂ zur Unterdrückung der Nebenreaktion von Substrat mit Hypochlorit benötigt wird. Ein zusätzlicher Nachteil ergibt sich durch das Anfallen stöchiometrischer Salzmengen.

Das Dokument WO 2009/033247 A2 beschreibt die Oxidation eines ätherischen Öls aus *laranja pera* (*citrus sinensis*), welches 96% Limonen enthält, mit einer 35%igen H₂0₂-Lösung. Dabei wird ein heterogener Katalysator der allgemeiner Formel L/MₓN_{y}, wobei M= Zr, Al, Si, Ti; N=O; x und y= 2 oder 3, und L= Co, Ti, V, Cr, Mn, Fe, Cu, Mo, W, Re. Nach dem beschriebenen Verfahren, wird die Reaktionsmischung bei einer Temperatur von 25 °C bis 125 °C für 24 Stunden erhitzt. Unter diesen Bedingungen wurde 40-65% des Limonens in oxygenierten Produkten - wie Carvon, Carveol, und Limonen-Epoxiden - verwandelt. Der genaue verwendete Katalysator wird im Dokument nicht angegeben. Nachteilig ist es in diesem Verfahren die Dauer der Oxidationsreaktion, die 24 Stunden beträgt. Somit lässt sich die Selektivität der Reaktion in Richtung die Bildung von Epoxy- und Peroxid-Derivaten, insbesondere die Bildung Peroxid-Derivaten schlecht steuern. In der Tat, das Dokument WO 2009/033247 A2 offenbart die Bildung von Peroxid-Derivaten des Limonens nicht.

Das Dokument US 3 014 047 A offenbart die Oxidation von d-Limonen zu Peroxid-Derivaten durch Luft bei einer Temperaturbereich von 25-80 °C. Nachteilig ist es wiederum die Dauer der Oxidationsreaktion. So beträgt die Dauer der Oxidationsreaktion etwa 5 Stunden bei 80 °C bis über sechs Tagen bei 25 °C. Die bessere Ergebnisse sind erhalten worden, wenn die Reaktion bei 80 °C für 30 Stunden. Versuche die Ergebnisse zu verbessern, bei Erhöhung der Reaktionszeit, schlugen jedoch fehlt, da die gebildeten Peroxid-Derivaten zur Zersetzung unter diesen Bedingungen neigen.

Es besteht daher ein starkes Bedürfnis nach neuen Verfahren zur Herstellung von oxidierten Derivaten von Limonen, insbesondere Epoxy- und Peroxid-Derivaten und ganz besonders bevorzugt Peroxid-Derivaten, die kürzere Reaktionsdauer benötigen und unter solchen Bedingungen ablaufen, so dass die Zersetzung der gewonnenen Verbindungen vermindert wird.

Die Aufgabe der vorliegenden Erfindung hat somit darin bestanden, ein industriell nutzbares Verfahren zur Herstellung von oxidierten Derivaten von Limonen, insbesondere Epoxy- und Peroxid-Derivaten, und ganz besonders bevorzugt Peroxid-Derivaten, zur Verfügung zu stellen, welches die eingangs geschilderten Nachteile des Standes der Technik verbessert. Dabei sollten die produzierten Verbindungen über vorteilhafte sensorische (geruchliche und geschmackliche) Eigenschaften verfügen, die Reaktion schnell, wirtschaftlich und im industriellen Maßstab ablaufen.

Eine zweite Aufgabe der vorliegenden Erfindung hat somit darin bestanden, ein industriell nutzbares Verfahren zur Herstellung von oxidierten Derivaten von Limonen, insbesondere Epoxy- und Peroxid-Derivaten, und ganz besonders bevorzugt Peroxid-Derivaten, zur Verfügung zu stellen, die sich in einer einfachen Art und Weise in verschiedensten Duft-, Aroma- oder Geschmacksstoffen weiter verarbeitet lassen.

### BESCHREIBUNG DER ERFINDUNG

Gegenstand der vorliegenden Erfindung ist ein Verfahren zur Oxidation von Limonen umfassend die Umsetzung von Limonen mit Wasserstoffperoxid in Gegenwart eines Katalysators enthaltend Atome oder/und Ionen mindestens eines Metalls ausgewählt aus der Gruppe von Molybdän, Wolfram, Scandium, Vanadium, Titan, Lanthan, Zirkon, Praseodym, Neodym, Samarium, Europium, Terbium, Dysprosium, Erbium oder Ytterbium, dadurch gekennzeichnet, dass das Molgewicht des Katalysators kleiner als 2000 g/mol ist und dass die Umsetzung bei einem pH-Wert größer als 7,5 erfolgt.

Überraschenderweise wurde gefunden, dass mittels der oben genannten Verfahren, oxidierten Derivaten von Limonen, insbesondere Epoxy- und Peroxid-Derivaten, und ganz besonders bevorzugt Peroxid-Derivaten, hergestellt werden können, so dass die Oxidationsreaktion in deutlich kürzere Reaktionszeiten abläuft als die von dem Stand der Technik beschriebenen Reaktionszeiten.

Darüber hinaus wurde überraschenderweise herausgestellt, dass die Durchführung der Reaktion bei einem pH-Wert größer als 7,5 die Stabilität der hergestellten Produkte erhöht und die Bildung von unerwünschten Nebenprodukten vermindert.

In einer bevorzugten Ausführungsform ist das Molgewicht des Katalysators kleiner als 1000 g/mol und insbesondere bevorzugt kleiner als 500 g/mol.

Die Metalle können auch in Form von beispielsweise Oxokomplexen, Oxiden, Hydroxiden, Salzen, wie z.B. Nitraten, Carboxylaten, Carbonaten, Chloriden, Fluoriden, Sulfaten oder Tetrafluoroboraten, vorliegen.

Die Katalysatoren können insbesondere Atome oder/und Ionen von Molybdän, Wolfram, Scandium, Vanadium, Titan und Lanthan und besonders bevorzugt von Molybdän und Wolfram enthalten.

Die Katalysatoren können insbesondere aus folgenden Verbindungen bestehen oder diese enthalten: Natriummolybdat, Natriummolybdat-Dihydrat, Natriumwolframat, Natriumwolframat-Dihydrat und Lanthannitrat. Die Katalysatoren können insbesondere als Feststoff oder in gelöster Form eingesetzt werden.

Die Reaktion kann insbesondere in mindestens einem organischen Lösungsmittel stattfinden. Beispielsweise können die Lösungsmittel aus der Gruppe der Alkohole mit 1 bis 8 C-Atomen und Amide ausgewählt werden. Auch können die Lösungsmittel aus der Gruppe von Methanol, Ethanol, Propanol, i-Propanol, Ethylenglykol, Propylenglykol, N-Methylformamid, Dimethylformamid oder N-Methylpyrrolidon ausgewählt werden. Optional kann das mindestens eine organische Lösungsmittel bis zu 30 Gew.-% Wasser enthalten.

Es hat sich herausgestellt, dass die Oxidationsreaktion besonders vorteilhaft bei einem pH-Wert größer als 8 abläuft, und weiter bevorzugt größer 9. Demzufolge erfolgt die Umsetzung von Limonen mit Wasserstoffperoxid in Gegenwart des Katalysators in einer bevorzugten Ausführungsform bei einem pH-Wert größer als 8 läuft, und weiter bevorzugt größer 9. Unter diesen Bedingungen wird die Bildung von Abbauprodukten des Limonens besonders günstig vermindert, insbesondere Abbauprodukte die aus Umlagerungen, Spaltung der Doppelbindungen und Dehydrierungen entstehen.

Es hat sich ebenso herausgestellt, dass die Oxidationsreaktion besonders vorteilhaft bei Temperaturen zwischen 25 und 90°C, bevorzugt zwischen 40 und 90°C abläuft.

In einer weiteren bevorzugten Ausführungsform erfolgt die Oxidationsreaktion bei einem pH-Wert größer als 8 und Temperaturen im Bereich 40 und 90°C, ganz besonders bevorzugt bei einem pH-Wert größer als 9 und Temperaturen im Bereich 40 und 90°C.

Weitere vorteilhafte Ergebnisse können bei einer Katalysatormenge von 1 bis 50 Mol-% bezogen auf Limonen oder / und 2-10 Moläquivalente Wasserstoffperoxid pro 1 Mol Limonen erzielt werden.

Als Produkte entstehen überwiegend die Hydroperoxide des Limonens. Somit ist auch ein Gegenstand der vorliegenden Erfindung die Verwendung des oben beschriebenen Verfahrens zur Herstellung einer Aromazusammensetzung umfassend mindestens ein Peroxid-Derivat, vorzugsweise zwei oder mehrere Peroxid-Derivate, des Limonens.

Die durch das Verfahren gemäß der vorliegenden Anmeldung gewonnene Mischung von oxidierten Derivaten können durch übliche Trennverfahren, beispielweise durch Gaschromatographie-Massenspektrometrie (GC-MS), und Hochleistungsflüssigkeitschromatographie (HPLC) oder fraktionierte Destillation erzielt werden.

Gegebenenfalls kann die gewonnene Mischung von oxidierten Derivaten, insbesondere Hydroperoxide des Limonens direkt weiter, d.h. ohne Isolierung, weiter verarbeitet werden, um wertvolle Duft-, Aroma- oder Geschmacksstoffen herzustellen.

So wird in einer weiteren Ausführungsform die durch das Oxidationsverfahren gemäß der vorliegenden Anmeldung erhaltene Mischung aus Hydroperoxiden des Limonens direkt mit einem Reduktionsmittel umgesetzt.

Somit ist auch ein Gegenstand der vorliegenden Erfindung ein Verfahren zur Herstellung von Hydroxyderivaten von Limonen, umfassend die folgenden Schritte:
(a) Oxidation von Limonen nach einem der vorhergehenden Ansprüche, und
(b) Umsetzung der im Schritt (a) gewonnenen Mischung mit einem Reduktionsmittel.

Das bevorzugte Reduktionsmittel ist Natriumsulfit.

Die Reduktion kann beispielsweise durch Einleitung der Reaktionsprodukte der Oxidation in eine wässrige Natriumsulfitlösung und anschließendem Rühren bei 25 bis 90 °C und einem pH-Wert größer als 7,5 erfolgen.

Im Anschluss wird die erhaltene Reaktionsmischung durch übliche Trennverfahren, wie etwa fraktionierte Destillation aufgetrennt. Aus der Reaktionsmischung lassen sich beispielweise p-2,8-Menthadien-1-ol, p-1(7),8-Menthadien-2-ol und Carveol gewinnen, wie veranschaulicht in den Beispielen der vorliegenden Anmeldung.

Die Umsetzungen können sowohl im Batch- als auch Konti-Verfahren durchgeführt werden. Während der Umsetzungen kann ein Stickstoffstrom durch die Reaktionsapparatur geleitet werden.

Bei einem möglichen jedoch nicht limitierenden Beispiel einer batchweisen Umsetzung werden Limonen, Methanol und der Katalysator, gegebenenfalls in Wasser gelöst, vorgelegt und bei der gewählten Reaktionstemperatur Wasserstoffperoxid-Lösung zudosiert. Nach dem Reaktionsende werden die gebildeten Hydroperoxide durch Eindosieren in eine Natriumsulfitlösung reduziert.

Bei einem möglichen jedoch nicht limitierenden Beispiel einer kontinuierlichen Durchführung werden die Einsatzstoffe in den unteren Bereich in eines Rohrreaktors eindosiert und die abreagierte Reaktionsmischung im oberen Bereich abgenommen bzw. in die Natriumsulfit-Lösung geleitet.

### GEWERBLICHE ANWENDBARKEIT

Gemäß der vorliegenden Erfindung können oxidierten Derivaten von Limonen hergestellt werden, insbesondere von Epoxy- und Peroxid-Derivaten, und ganz besonders bevorzugt Peroxid-Derivaten, die selbst als Duft-, Aroma- oder Geschmacksstoffen umgesetzt bzw. zum wertwollen Duft-, Aroma- oder Geschmacksstoffen weiter verarbeitet werden können.

### BEISPIELE

Die vorliegende Erfindung wird unter Bezugnahme auf die nachstehenden Beispiele leichter zu verstehen sein. Jedoch dienen diese Beispiele lediglich zur Veranschaulichung der Erfindung und können nicht als einschränkend in Bezug auf den Schutzbereich der Erfindung ausgelegt werden.

### BEISPIEL 1

In einer 100 mL-Dreihalskolbenrührapparatur werden 2,72 g D-Limonen in 24,4 g Methanol vorgelegt. Hierzu werden 0,22 g Natriummolybdat-Dihydrat, gelöst in 1,86 g Wasser, zugegeben und mit 5%iger Natronlauge auf pH = 10 eingestellt. Es wird auf Rückflusstemperatur erhitzt und 4 g Wasserstoffperoxid 50%ig innerhalb von 30 Minuten zudosiert und weitere 5 Minuten nachreagieren lassen. Das Reaktionsgemisch wird anschließend bei 60°C in eine Lösung von 2,5 g Natriumsulfit in 7,2 g Wasser gegeben und zur vollständigen Peroxidzersetzung 3 Stunden nachgerührt. Es wird vom ausgefallenen Niederschlag abfiltriert und das Filtrat am Rotationsverdampfer eingeengt. Die Rohproduktzusammensetzung nach GC-Flächen-% ist in Tabelle 1 angegeben.

### BEISPIEL 2

In einer 100 ml-Dreihalskolbenrührapparatur werden 2,72 g D-Limonen in 11 g Methanol und 7 g Wasser vorgelegt. Die weitere Durchführung erfolgte analog Beispiel 1.

### BEISPIEL 3

Analog Beispiel 2, jedoch mit 0,3 g Natriumwolframat Dihydrat, gelöst in 1,86g Wasser.

### BEISPIEL 4

Analog Beispiel 1, jedoch mit 0,4 g Lanthannitrat, gelöst in 1,86 g Wasser.

In Tabelle 1 ist erkennbar, dass Ausbeute der hergestellten Mengen der Verbindungen p-2,8-Menthadien-1-ol, p-1(7),8-Menthadien-2-ol und Carveol in Summe sehr hoch ist. Außerdem lässt erkennen, dass bei den unterschiedlichen Herstellungsvarianten das Verhältnis der Stoffe untereinander variiert.

### BEISPIEL 5

In einer 1L-Doppelmantelrührapparatur werden 27,2 g D-Limonen in 244 g Methanol vorgelegt. Hierzu werden 4,4 g Natriummolybdat-Dihydrat, gelöst in 18,6 g Wasser, gegeben und mit 1,5 g 5%iger Natronlauge auf pH = 10 eingestellt. Es wird auf Rückflusstemperatur erhitzt und 40,8 g Wasserstoffperoxid 50%ig innerhalb von 30 Minuten zudosiert und weitere 5 Minuten nachreagieren lassen. Das Reaktionsgemisch wird anschließend bei 60°C in eine Lösung von 25 g Natriumsulfit in 72 g Wasser gegeben und zur vollständigen Peroxidzersetzung 3 Stunden bei 60°C nachgerührt. Es wird vom ausgefallenen Niederschlag abfiltriert und mit tert.-Butylmethylether nachgespült. Vom erhaltenen Filtrat werden Methanol und tert.-Butylmethylether abdestilliert. Mit dem verbleibenden 2-phasigen Rückstand wird eine Phasentrennung durchgeführt und die wässrige Phase 2 Mal mit je 20 g tert.-Butylmethylether extrahiert. Die vereinigten organischen Phasen werden mittels Rotationsverdampfer eingeengt und der verbleibende Rückstand im Vakuum destilliert. Es werden 13,1 g Destillat erhalten.

### BEISPIEL 6 (kontinuierliche Verfahrensweise)

In ein Doppelmantelreaktionsrohr mit einem Volumen von 150 ml werden13,6 g D-Limonen, 61 g Methanol und 1,1 g Natriummolybdat-Dihydrat, gelöst in 2 g Wasser, gegeben und 0,75 g 5%ige Natronlauge zugefügt. Das Reaktionsgemisch wird auf 60°C erwärmt und innerhalb von 30 Minuten werden hierzu 20,4 g Wasserstoffperoxid 50%ig in den unteren Bereich des Reaktors dosiert. Anschließend werden innerhalb von 45 Minuten mittels Dosierpumpen parallel 61,2 g Wasserstoffperoxid 50%ig sowie eine gerührte Mischung bestehend aus 40,8 g D-Limonen, 183 g Methanol, 3,3 g Natriummolybdat-Dihydrat, 18 g Wasser und 2,3 g 5%ige Natronlauge in den Bodenbereich des Reaktors dosiert. Die resultierende Reaktionsmischung wird aus dem oberen Reaktorbereich kontinuierlich in eine Lösung aus 50 g Natriumsulfit und 144 g Wasser geleitet und bei 60°C für 3 Stunden gerührt. Die weitere Aufarbeitung erfolgt analog Beispiel 5. Es werden 29,1 g Destillat erhalten.

**Tabelle 1**

| Anteile in der Reaktionsmischung nach dem Reduktionsschritt | | | | | |
|---|---|---|---|---|---|
| **Beispiel** | **GC-Flächen-%** | | | | |
| | D-Limonen | cis/trans p-2,8-Menthadien-1-ol | cis/trans p-1(7),8-Menthadien-2-ol | cis/trans Carveol | Summe der Produkte |
| | | (Produkt A) | (Produkt B) | (Produkt C) | A, B und C |
| 1 | 5,8 | 35,6 | 36 | 14,7 | 86,3 |
| 2 | 2,9 | 31,9 | 39 | 19,3 | 90,2 |
| 3 | 14,6 | 11,9 | 27,5 | 25,8 | 65,2 |
| 4 | 15 | 18,5 | 36,3 | 24,9 | 79,7 |
| 5 | n.n. | 40,9% | 38,5% | 17,8% | 97,2 |
| 6 | 27,7% | 28,2% | 29% | 11,5% | 68,7 |

### Analyse des cis/trans-Gehaltes von p-2,8-Menthadien-1-ol

Der Gehalt der cis / trans Isomere wird gaschromatographisch bestimmt. Das Verhältnis aus cis p-2,8-Menthadien-1-ol zu trans p-2,8-Menthadien-1-ol beträgt etwa 1:3 und entspricht damit annähernd dem Verhältnis aus Schenk et. al, Liebigs Ann. Chemie, 674 (1964), 93-117. Die Verhältnisse von cis zu trans p-1(7),8-Menthadien-2-ol sowie von cis zu trans Carveol entsprechen ebenfalls den in Schenk et al. angegebenen Verhältnissen. Die Isomerenverhältnisse sind somit wie beim Photooxidationsverfahren.

## Patentansprüche

1. Verfahren zur Oxidation von Limonen umfassend die Umsetzung von Limonen mit Wasserstoffperoxid in Gegenwart eines Katalysators enthaltend Atome oder/und Ionen mindestens eines Metalls ausgewählt aus der Gruppe von Molybdän, Wolfram, Scandium, Vanadium, Titan, Lanthan, Zirkon, Praseodym, Neodym, Samarium, Europium, Terbium, Dysprosium, Erbium oder Ytterbium, **dadurch gekennzeichnet, dass** das Molgewicht des Katalysators kleiner als 2000 g/mol ist und dass die Umsetzung bei einem pH-Wert größer als 7,5 erfolgt.

2. Verfahren nach Anspruch 1, **dadurch gekennzeichnet, dass** der Katalysator Atome oder/und Ionen mindestens eines Metalls ausgewählt aus der Gruppe von Molybdän, Wolfram, Scandium, Vanadium, Titan und Lanthan und bevorzugt von Molybdän und Wolfram enthält.

3. Verfahren nach einem der vorhergehenden Ansprüche, **dadurch gekennzeichnet, dass** der Katalysator ausgewählt ist aus der Gruppe von Natriummolybdat, Natriummolybdat-Dihydrat, Natriumwolframat, Natriumwolframat-Dihydrat und Lanthannitrat.

4. Verfahren nach einem der vorhergehenden Ansprüche, **dadurch gekennzeichnet, dass** die Reaktion in mindestens einem organischen Lösungsmittel stattfindet.

5. Verfahren nach Anspruch 4, **dadurch gekennzeichnet, dass** die Lösungsmittel aus der Gruppe bestehend aus Alkohole mit 1 bis 8 C-Atomen und Amide ausgewählt werden.

6. Verfahren nach einem der vorhergehenden Ansprüche, **dadurch gekennzeichnet, dass** der pH-Wert größer als 8 ist.

7. Verfahren nach einem der vorhergehenden Ansprüche, **dadurch gekennzeichnet, dass** die Temperatur von 25 bis 90°C beträgt.

8. Verfahren nach einem der vorhergehenden Ansprüche, **dadurch gekennzeichnet, dass** der pH-Wert größer als 8 ist und die Temperatur im Bereich 40 °C bis 90 °C liegt.

9. Verfahren nach einem der vorhergehenden Ansprüche, **dadurch gekennzeichnet, dass** der pH-Wert größer als 9 ist und die Temperatur im Bereich 40 °C bis 90 °C liegt.

10. Verfahren nach einem der vorhergehenden Ansprüche, **dadurch gekennzeichnet, dass** die verwendete Katalysatormenge 1 bis 50 Mol-% bezogen auf Limonen beträgt.

11. Verfahren nach einem der vorhergehenden Ansprüche, **dadurch gekennzeichnet, dass** 2-10 Moläquivalente Wasserstoffperoxid pro 1 Mol Limonen verwendet werden.

12. Verfahren zur Herstellung von Hydroxyderivaten von Limonen, umfassend die folgenden Schritte:
(a) Oxidation von Limonen nach einem der vorhergehenden Ansprüche, und
(b) Umsetzung der im Schritt (a) gewonnenen Mischung mit einem Reduktionsmittel.

13. Verfahren nach Anspruch 12, **dadurch gekennzeichnet, dass** es bei 25 bis 90°C, bei einem pH-Wert größer als 7,5 durchgeführt wird.

14. Verfahren umfassend das Auftrennen eines Reaktionsproduktes erhalten oder erhältlich nach einem der Ansprüche 12 oder 13, bevorzugt durch Destillation.

15. Verwendung des Verfahrens nach einem der Ansprüche 1 bis 11 zur Herstellung einer Aromazusammensetzung umfassend mindestens ein Peroxid-Derivat, vorzugsweise zwei oder mehrere Peroxid-Derivate, des Limonens.

## Claims

1. A process for the oxidation of limonene, comprising the reaction of limonene with hydrogen peroxide in the presence of a catalyst containing atoms and/or ions of at least one metal, selected from the group consisting of molybdenum, tungsten, scandium, vanadium, titanium, lanthanum, zirconium, praseodymium, neodymium, samarium, europium, terbium, dysprosium, erbium or ytterbium, **characterised in that** the molecular weight of the catalyst is less than 2,000 g/mol and that the reaction is performed at a pH value of more than 7.5.

2. The process of claim 1, **characterised in that** the catalyst contains atoms and/or ions of at least one metal, selected from the group consisting of molybdenum, tungsten, scandium, vanadium, titanium and lanthanum, and preferably of molybdenum and tungsten.

3. The process of any one of the preceding claims, **characterised in that** the catalyst is selected from the group consisting of sodium molybdate, sodium molybdate dihydrate, sodium tungstate, sodium tungstate dihydrate and lanthanum nitrate.

4. The process of any one of the preceding claims, **characterised in that** the reaction is performed in at least one organic solvent.

5. The process of claim 4, **characterised in that** the solvents are selected from the group consisting of C1-C8 alcohols and amides.

6. The process of any one of the preceding claims, **characterised in that** the pH value is more than 8.

7. The process of any one of the preceding claims, **characterised in that** the temperature is from 25 to 90°C.

8. The process of any one of the preceding claims, **characterised in that** the pH value is more than 8, and the temperature is in the range from 40 °C to 90 °C.

9. The process of any one of the preceding claims, **characterised in that** the pH value is more than 9, and the temperature is in the range from 40 °C to 90 °C.

10. The process of any one of the preceding claims, **characterised in that** the amount of catalyst used is 1 to 50 mole percent, based on limonene.

11. The process of any one of the preceding claims, **characterised in that** 2-10 molar equivalents of hydrogen peroxide per 1 mole limonene are used.

12. A process for the production of hydroxy derivatives of limonene, comprising the following steps:
(a) oxidation of limonene according to any one of the preceding claims, and
(b) reaction of the mixture obtained in step (a) with a reducing agent.

13. The process of claim 12, **characterised in that** it is performed at a temperature of 25 to 90°C at a pH value of more than 7.5.

14. A process, comprising separating a reaction product, obtained or obtainable according to any one of claims 12 or 13, preferably, by distillation.

15. Use of the process of any one of claims 1 to 11 for the production of an aroma composition, comprising at least one peroxide derivative, preferably two or more peroxide derivatives, of limonene.

## Revendications

1. Procédé d'oxydation de limonène, comprenant la mise en réaction de limonène avec du peroxyde d'hydrogène en présence d'un catalyseur contenant des atomes et/ou des ions d'au moins un métal choisi dans le groupe constitué par le molybdène, le tungstène, le scandium, le vanadium, le titane, le lanthane, le zirconium, le praséodyme, le néodyme, le samarium, l'europium, le terbium, le dysprosium, l'erbium ou l'ytterbium, **caractérisé en ce que** le poids moléculaire du catalyseur est inférieur à 2 000 g/mol et **en ce que** la réaction a lieu à un pH supérieur à 7,5.

2. Procédé selon la revendication 1, **caractérisé en ce que** le catalyseur contient des atomes et/ou des ions d'au moins un métal choisi dans le groupe constitué par le molybdène, le tungstène, le scandium, le vanadium, le titane et le lanthane, et de préférence de molybdène et de tungstène.

3. Procédé selon l'une quelconque des revendications précédentes, **caractérisé en ce que** le catalyseur est choisi dans le groupe constitué par le molybdate de sodium, le molybdate de sodium dihydraté, le tungstate de sodium, le tungstate de sodium dihydraté et le nitrate de lanthane.

4. Procédé selon l'une quelconque des revendications précédentes, **caractérisé en ce que** la réaction a lieu dans au moins un solvant organique.

5. Procédé selon la revendication 4, **caractérisé en ce que** les solvants sont choisis dans le groupe constitué par les alcools contenant 1 à 8 atomes C et les amides.

6. Procédé selon l'une quelconque des revendications précédentes, **caractérisé en ce que** le pH est supérieur à 8.

7. Procédé selon l'une quelconque des revendications précédentes, **caractérisé en ce que** la température est de 25 à 90 °C.

8. Procédé selon l'une quelconque des revendications précédentes, **caractérisé en ce que** le pH est supérieur à 8 et la température se situe dans la plage allant de 40 °C à 90 °C.

9. Procédé selon l'une quelconque des revendications précédentes, **caractérisé en ce que** le pH est supérieur à 9 et la température se situe dans la plage allant de 40 °C à 90 °C.

10. Procédé selon l'une quelconque des revendications précédentes, **caractérisé en ce que** la quantité de catalyseur utilisée est de 1 à 50 % en moles, par rapport au limonène.

11. Procédé selon l'une quelconque des revendications précédentes, **caractérisé en ce que** 2 à 10 équivalents molaires de peroxyde d'hydrogène sont utilisés pour 1 mole de limonène.

12. Procédé de fabrication de dérivés hydroxy de limonène, comprenant les étapes suivantes :
(a) l'oxydation de limonène selon l'une quelconque des revendications précédentes, et
(b) la mise en réaction du mélange obtenu à l'étape (a) avec un réducteur.

13. Procédé selon la revendication 12, **caractérisé en ce qu'**il est réalisé à une température de 25 à 90 °C, à un pH supérieur à 7,5.

14. Procédé comprenant la séparation d'un produit de réaction obtenu ou pouvant être obtenu selon l'une quelconque des revendications 12 ou 13, de préférence par distillation.

15. Utilisation du procédé selon l'une quelconque des revendications 1 à 11 pour la fabrication d'une composition d'arôme comprenant au moins un dérivé de peroxyde, de préférence deux dérivés de peroxyde ou plus, de limonène.
